# EUROPÄISCHE PATENTSCHRIFT

(11) **EP 3 750 472 B1**
(45) Veröffentlichungstag und Bekanntmachung des Hinweises auf die Patenterteilung: **19.04.2023**
(21) Anmeldenummer: 19179895.8
(22) Anmeldetag: 13.06.2019
(51) Int. Cl.: A61B 5/00, A61M 25/00

(54) **KATHETEREINRICHTUNG ZUM PLATZIEREN EINES SENSORS**
CATHETER DEVICE FOR PLACING A SENSOR
DISPOSITIF CATHÉTER PERMETTANT DE POSER UN CAPTEUR

(43) Veröffentlichungstag der Anmeldung: 16.12.2020
(73) Patentinhaber: BIOTRONIK SE & Co. KG, 12359 Berlin (DE)
(72) Erfinder: Kaufmann, Ralf, 79540 Lörrach (DE)
(74) Vertreter: Biotronik Corporate Services SE

(56) Entgegenhaltungen:
- US-A1- 2012 108 986
- US-A1- 2015 273 212
- US-A1- 2016 279 423

## Beschreibung

Die Erfindung betrifft eine Kathetereinrichtung zum Platzieren einer Sensoreinrichtung sowie ein System mit einer Kathetereinrichtung und einer Sensoreinrichtung.

Eine derartige Kathetereinrichtung umfasst einen Schaft und eine an dem Schaft angeordnete Kopplungseinrichtung zum Koppeln der Kathetereinrichtung mit der zumindest ein Bügelelement aufweisenden Sensoreinrichtung.

Eine solche Kathetereinrichtung kann beispielsweise als Absetzkatheter zum Platzieren einer Sensoreinrichtung im menschlichen Herzen oder in einem anderen Blutgefäß verwendet werden. Eine Sensoreinrichtung der hier in Rede stehenden Art kann beispielsweise als Drucksensor, Temperatursensor, Sauerstoffsensor oder Flusssensor ausgebildet sein und soll innerhalb eines Patienten Messdaten aufnehmen und beispielsweise mittels Telemetrie an ein externes Gerät übertragen. Solche Sensoreinrichtungen können beispielsweise im Rahmen einer Heimüberwachung (sogenanntes Home Monitoring) von schwer kranken Patienten eingesetzt werden, um eine Überwachung und Versorgung von Patienten in einer nicht stationären Umgebung zu ermöglichen.

Wünschenswert sind Sensoreinrichtungen, die atraumatisch, also schonend für eine Gefäßwand, in einem Patienten platzierbar sind. Das Platzieren soll hierbei einfach, flexibel und zuverlässig mittels der Kathetereinrichtung möglich sein.

Implantierbare Sensoreinrichtungen zur Drucküberwachung im menschlichen Herzen sind beispielsweise in den Artikeln J.H. Dreyfuss et al., "Prevent the Next Heart Attack: Implant a CardioMEMS Device in a Heart Failure Patient, " M.D./alert, Feb. 2016; P. B. Adamson et al., "Champion* Trial Rational and Design: The Long-Term Safety and Clinical Efficacy of a Wireless Pulmonary Artery Pressure Monitoring System", Journal of Cardiac Failure, Vol. 17, No. 1, 2011; und E. Y. Chow, "Fully Wireless Implantable Cardiovascular Pressure Monitor Integrated with a Medical Stent", IEEE Transactions on Biomedical Engineering, Vol. 57, No. 6, 2010 beschrieben. US 2012/108986 A1 beschreibt ein implantierbares medizinisches Gerät, wie beispielsweise ein Sensor zum Überwachen eines ausgewählten intern erfassbaren physiologischen Parameters eines Patienten, das an einem Befestigungselement angebracht ist und das im Patienten einsetzbar ist, um den Sensor zu positionieren und auszurichten, damit er seine Funktion ausführen kann. US 2016/279423 A1 beschreibt Techniken und Systeme zum Abgeben einer implantierbaren medizinischen Vorrichtung. US 2015/273212 A1 beschreibt ein Werkzeug eines interventionellen medizinischen System, das einen Kern umfasst, der dazu konfiguriert ist, vorübergehend an der implantierbaren medizinischen Vorrichtung befestigt zu werden, wenn das Werkzeug die Vorrichtung entfaltet, um ein Befestigungselement der Vorrichtung zum Eingriff mit Gewebe an einer Zielimplantationsstelle freizulegen.

Aufgabe ist es, eine Kathetereinrichtung zum Platzieren einer Sensoreinrichtung sowie ein System mit einer Kathetereinrichtung und einer Sensoreinrichtung zur Verfügung zu stellen, die ein einfaches, flexibles und zuverlässiges Platzieren einer Sensoreinrichtung ermöglichen. Gegebenenfalls soll ein erneutes Greifen der Sensoreinrichtung zum Korrigieren der Sensorlage oder zum Entfernen der Sensoreinrichtung ermöglicht werden.

Diese Aufgabe wird durch einen Gegenstand mit den Merkmalen des Anspruchs 1 gelöst.

Demnach weist die Kopplungseinrichtung ein Kopplungselement und ein zu dem Kopplungselement verstellbares Verriegelungselement auf. Das Kopplungselement weist zumindest einen Eingriffsabschnitt auf, mit dem das zumindest eine Bügelelement der Sensoreinrichtung in Eingriff bringbar ist, wobei das Verriegelungselement ausgebildet ist, in einer gekoppelten Stellung das zumindest eine Bügelelement an dem zumindest einen Eingriffsabschnitt zu sperren, und zu dem Kopplungselement verstellbar ist, um das zumindest eine Bügelelement zum Lösen von dem zumindest einen Eingriffsabschnitt freizugeben.

Die Kathetereinrichtung verwendet eine Kopplungseinrichtung, um eine Kopplung zu einer zu implantierenden Sensoreinrichtung herzustellen. Über die Kopplungseinrichtung kann die Sensoreinrichtung gegriffen werden, sodass in einer gekoppelten Stellung die Sensoreinrichtung fest mit der Kathetereinrichtung verbunden ist.

Die Kopplung wird hierbei dadurch hergestellt, dass die Sensoreinrichtung in der gekoppelten Stellung mit einem oder mehreren Bügelelementen in einen oder mehrere Eingriffsabschnitte eines Kopplungselements der Kopplungseinrichtung eingreift. Die Eingriffsabschnitte können beispielsweise nach Art von Hinterschnitten geformt sein und somit eine formschlüssige Verbindung zwischen der Sensoreinrichtung und der Kopplungseinrichtung herstellen. Die formschlüssige Verbindung ist dabei über ein Verriegelungselement derart gesperrt, dass die Bügelelemente der Sensoreinrichtung in der gekoppelten Stellung nicht ohne weiteres, jedenfalls nicht ohne Betätigung des Verriegelungselements, außer Eingriff von den Eingriffsabschnitten des Kopplungselements gelangen können, sondern über die Kopplungseinrichtung zuverlässig und belastbar an der Kathetereinrichtung gehalten sind.

In der gekoppelten Stellung kann die Sensoreinrichtung mittels der Kathetereinrichtung in einem Patienten implantiert werden und dazu an einen vorbestimmten Ort im menschlichen Herzen oder in einem anderen Gefäß gebracht werden. Ist ein Bestimmungsort erreicht, kann das Verriegelungselement betätigt werden, um die Bügelelemente der Sensoreinrichtung freizugeben und somit ein Lösen der Sensoreinrichtung von der Kopplungseinrichtung und somit von der Kathetereinrichtung zu ermöglichen.

Das Aufheben der Kopplung kann durch Verstellen des Verrieglungselements mit vergleichsweise kleinem Hub erfolgen. Durch Verstellen des Verriegelungselements wird der zumindest eine Eingriffsabschnitt des Kopplungselements freigegeben, sodass das zumindest eine Bügelelement der Sensoreinrichtung in einfacher Weise außer Eingriff von dem Eingriffsabschnitt gebracht werden kann.

Das Verriegelungselement dient dazu, das zumindest eine Bügelelement, wenn es in Eingriff mit dem zumindest einen Eingriffsabschnitt des Kopplungselements ist, so zu sperren, dass das zumindest eine Bügelelement nicht außer Eingriff von dem Eingriffsabschnitt gelangen kann. Das Verriegelungselement ist hierzu in einer Sperrstellung in einer derartigen räumlichen Lagebeziehung zu dem Eingriffsabschnitt, dass das Bügelelement nicht aus dem Eingriffsabschnitt entfernt werden kann. Erst wenn das Verriegelungselement zum Lösen der Kopplung betätigt ist, kann das Bügelelement aus dem zugeordneten Eingriffsabschnitt entfernt und somit die Sensoreinrichtung von der Kathetereinrichtung entkoppelt werden.

Die Kopplungseinrichtung kann mit vergleichsweise kleinem Bauraum aufgebaut werden, was es ermöglicht, eine Kathetereinrichtung mit dünnem Schaft (Durchmesser zum Beispiel 6F oder 7F oder 1,8 mm bis 2,4 mm) zu verwenden.

Das Kopplungselement ist, in einer Ausgestaltung, fest mit einem Abschnitt des Schafts, beispielsweise im Bereich eines distalen Endes des Schafts, verbunden. Der zumindest eine Eingriffsabschnitt ist zum Herstellen der Kopplung an dem Kopplungselement geformt derart, dass die Sensoreinrichtung über das zumindest eine Bügelelement in einfacher und zuverlässiger Weise in Eingriff mit dem Eingriffsabschnitt gebracht werden kann und in einer gekoppelten Stellung über das Verriegelungselement zu dem Kopplungselement verriegelt ist.

Das Verriegelungselement ist, in einer Ausgestaltung, beispielsweise entlang einer Betätigungsrichtung linear zu dem Kopplungselement verstellbar. Das Kopplungselement kann zum Verriegelungselement entlang der Betätigungsrichtung geführt sein, sodass das Verriegelungselement zwischen einer Sperrstellung zum Sperren des zumindest einen Bügelelements in Eingriff mit dem zumindest einen Eingriffsabschnitt des Kopplungselements und einer Lösestellung zum Freigeben der Sensoreinrichtung verstellbar ist.

In einer Ausgestaltung weist das Kopplungselement einen von dem Schaft abliegenden, distalen Rand auf, der sich beispielsweise entlang einer quer zur Längserstreckungsrichtung des Schafts gerichteten Ebene erstreckt und umlaufend an dem zum Beispiel hülsenförmig ausgebildeten Kopplungselement geformt ist. In den Rand ist, in einer Ausgestaltung, zumindest eine Aussparung eingeformt, an der der zumindest eine Eingriffsabschnitt nach Art eines Hinterschnitts geformt ist. Durch Eingriff in die Aussparung und den an der Aussparung geformten Eingriffsabschnitt kann ein jedes Bügelelement der Sensoreinrichtung somit mit dem Kopplungselement gekoppelt werden, wobei in der gekoppelten Stellung der Eingriff des Bügelelements in den Eingriffsabschnitt des Kopplungselements über das Verriegelungselement derart gesperrt ist, dass das Bügelelement nicht ohne weiteres - jedenfalls nicht ohne Betätigung des Verriegelungselements - außer Eingriff von dem Eingriffsabschnitt gebracht werden kann.

In einer Ausgestaltung weist das Verriegelungselement zumindest eine Eingriffsnut zum Aufnehmen des zumindest einen Bügelelements in der gekoppelten Stellung auf. Das Verriegelungselement kann beispielsweise einen in dem hülsenförmigen Kopplungselement einliegenden Körper aufweisen, der mit einer distalen Stirnseite in distaler Richtung nach außen weist. An dieser distalen Stirnseite können eine oder mehrere Eingriffsnuten geformt sein derart, dass in der gekoppelten Stellung der Sensoreinrichtung ein jedes Bügelelement der Sensoreinrichtung in eine Eingriffsnut des Verriegelungselements eingreift. Die Eingriffsnuten können sich beispielsweise radial zur (linear gerichteten) Betätigungsrichtung erstrecken und fluchten in der gekoppelten Stellung der Sensoreinrichtung derart mit dem Eingriffsabschnitt, dass ein jedes Bügelelement durch Eingriff in die Eingriffsnut zu dem jeweils zugeordneten Eingriffsabschnitt gesperrt ist und somit nicht außer Eingriff von dem Eingriffsabschnitt gebracht werden kann.

In einer Ausgestaltung weist die Kopplungseinrichtung ein mit dem Verriegelungselement verbundenes, entlang des Schafts erstrecktes Zugelement zum Verstellen des Verrieglungselements auf. Das Zugelement dient zum Betätigen des Verriegelungselements und kann auf Zug belastet werden, um auf diese Weise das Verriegelungselement in die Betätigungsrichtung zu dem Kopplungselement zu verstellen. Das Zugelement kann sich beispielsweise von der Kopplungseinrichtung bis zu hin zu einem am proximalen Ende des Schafts angeordneten Griff erstrecken und mit einem Betätigungselement des Griffs gekoppelt sein derart, dass durch Betätigen des Betätigungselements eine Zugkraft auf das Zugelement ausgeübt und darüber das Verriegelungselement in die Betätigungsrichtung relativ zu dem Kopplungselement der Kopplungseinrichtung verstellt werden kann.

Während die Kopplungseinrichtung zum Koppeln mit der Sensoreinrichtung am in den Körper eines Patienten einzuführenden distalen Ende des Schafts der Kathetereinrichtung angeordnet ist, ist der Griff am proximalen Ende des Schafts der Kathetereinrichtung angeordnet und somit außerhalb des Körpers des Patienten betätigbar, um nach bestimmungsgemäßer Platzierung der Sensoreinrichtung im Patienten die Kopplung zwischen der Sensoreinrichtung und der Kopplungseinrichtung aufzuheben oder, gegebenenfalls, zum Korrigieren der Lage der Sensoreinrichtung oder zum Entfernen der Sensoreinrichtung aus dem Patienten die Sensoreinrichtung erneut zu greifen. Beispielsweise kann das Betätigungselement zum Einwirken auf das Zugelement zum Verstellen des Verriegelungselements an dem Griff verdrehbar sein, wobei der Griff beispielsweise eine Verdrehsicherung aufweist, die ein unbeabsichtigtes Betätigen des Betätigungselements und somit ein unbeabsichtigtes Lösen vor bestimmungsgemäßer Platzierung der Sensoreinrichtung verhindert.

Die Kopplungseinrichtung weist ein an dem Kopplungselement angeordnetes, zwischen dem Kopplungselement und dem Verriegelungselement wirkendes elastisches Element auf. Das elastische Element kann mit einem an dem Körper des Verriegelungselements geformten Konusabschnitt wechselwirken, derart, dass bei einem Verstellen des Verriegelungselements in die Betätigungsrichtung zum Aufheben der Kopplung der Sensoreinrichtung mit der Kopplungseinrichtung das elastische Element in elastischer Weise verformt wird und eine elastische Gegenspannung entgegen der Betätigungsrichtung auf das Verriegelungselement ausübt. Auf diese Weise kann eine selbsttätige Zurückstellung des Verriegelungselements nach erfolgter Betätigung bewirkt werden, sodass das Verriegelungselement selbsttätig in eine der Kopplungsstellung entsprechende Ausgangsstellung zurück gelangt, wenn keine Zugkräfte mehr auf das Zugelement ausgeübt werden.

Das elastische Element kann - zusätzlich oder alternativ zum Bereitstellen einer elastischen Vorspannung - zum Abdichten eines Übergangs zwischen dem Kopplungselement und dem Schaft der Kathetereinrichtung dienen. Beispielsweise kann das elastische Element an einem Bodenabschnitt des Kopplungselements angeordnet sein, der sich quer zur Betätigungsrichtung erstreckt und einen Aufnahmeraum des Kopplungselements, in dem das Verriegelungselement in verstellbarer Weise aufgenommen ist, von dem Schaft der Kathetereinrichtung trennt. Der Bodenabschnitt kann hierbei eine Öffnung aufweisen, durch die hindurch das Zugelement zur Betätigung des Verriegelungselements geführt ist, wobei das elastische Element zum Beispiel nach Art einer Hülse derart im Bereich der Öffnung an dem Bodenabschnitt angeordnet sein kann, dass ein Übergang zwischen dem Bodenabschnitt und dem Verriegelungselement und somit die Öffnung in dem Bodenabschnitt, durch die das Zugelement hindurchgeführt ist, feuchtigkeitsdicht abgedichtet sind.

Das Verriegelungselement ist dem Bodenabschnitt mit einer Seite zugewandt, die der die zumindest eine Eingriffsnut tragenden, distalen Stirnseite des Verriegelungselements abgewandt ist. Bei einem Betätigen des Verriegelungselements zum Aufheben der Kopplung zwischen der Sensoreinrichtung und der Kopplungseinrichtung wird das Verriegelungselement dem Bodenabschnitt angenähert, wobei das elastische Element derart zwischen dem Verriegelungselement und dem Bodenabschnitt des Kopplungselements angeordnet ist, dass das elastische Element bei einem Verstellen des Verriegelungselements in die Betätigungsrichtung elastisch verformt wird, dabei aber vorzugsweise eine feuchtigkeitsdichte Abdichtung zwischen dem Verriegelungselement und dem Bodenabschnitt aufrechterhält.

Der Bodenabschnitt kann zudem als Anschlag für das Verriegelungselement dienen und somit einen maximalen Betätigungsweg für das Verriegelungselement definieren.

In einer Ausgestaltung weist der Schaft ein deflektierbares Schaftsegment auf, an dem die Kopplungseinrichtung angeordnet ist. Das deflektierbare Schaftsegment ist im Bereich des distalen Endes des Schafts geformt und kann so ausgelenkt werden, dass die Kathetereinrichtung in flexibler, steuerbarer Weise auch in anatomisch gewundene Gefäße, zum Beispiel bis hin zur Pulmonalarterie, geführt werden kann, um die Sensoreinrichtung zu platzieren.

Das deflektierbare Schaftsegment kann beispielsweise über dasselbe Betätigungselement am Griff betätigt werden, über das auch das Zugelement der Kopplungseinrichtung betätigt werden kann. Während die Betätigung des Zugelements zum Verstellen des Verriegelungselements beispielsweise durch Verdrehen des Betätigungselements am Griff erfolgen kann, kann eine Auslenkung des deflektierbaren Schaftsegments beispielsweise durch lineare Verstellung des Betätigungselements am Griff vorgenommen werden.

Ein System umfasst eine Kathetereinrichtung nach der vorangehend beschriebenen Art und eine mit der Kopplungseinrichtung der Kathetereinrichtung zu koppelnde Sensoreinrichtung. Eine solche Sensoreinrichtung kann beispielsweise als Drucksensor, Temperatursensor, Sauerstoffsensor oder Flusssensor ausgebildet sein und umfasst beispielsweise eine eigene Energieversorgung, zum Beispiel eine Batterie, sowie eine Kommunikationseinrichtung, zum Beispiel zur Datenübertragung mittels Telemetrie, sodass die Sensoreinrichtung im menschlichen Patienten in implantiertem Zustand in autarker Weise Überwachungsaufgaben übernehmen und Datensignale an eine externe Einrichtung übermitteln kann.

Eine solche Sensoreinrichtung umfasst beispielsweise ein Sensorgehäuse und eine oder mehrere an dem Sensorgehäuse angeordnete Bügelelemente, die in der gekoppelten Stellung mit zugeordneten Eingriffsabschnitten des Kopplungselements in Eingriff stehen und somit eine feste (aber lösbare) Verbindung zwischen der Sensoreinrichtung und der Kathetereinrichtung herstellen. Die Bügelelemente sind vorzugsweise aus einem superelastischen Material geformt und erstrecken sich derart an dem Sensorgehäuse, dass die Sensoreinrichtung in atraumatischer Weise, also gefäßwandschonend, in einem Gefäß platziert werden kann.

Beispielsweise kann ein jedes Bügelelement mit einem ersten Ende an einer distalen Seite des Sensorgehäuses und mit einem zweiten Ende an einer der distalen Seite abgewandten, proximalen Seiten des Sensorgehäuses befestigt sein. Das Bügelelement ist somit mit beiden Enden fest mit dem Sensorgehäuse verbunden und bildet eine von dem Sensorgehäuse vorstehende Schlaufe aus. Insbesondere im Bereich des proximalen Endes des Sensorgehäuses kann die Kopplungseinrichtung an den Bügelelementen angreifen, um eine Verbindung zwischen der Sensoreinrichtung und der Kathetereinrichtung herzustellen.

Beispielsweise kann die Sensoreinrichtung drei gleich gestaltete Bügelelemente aufweisen, die gleichverteilt um das Sensorgehäuse angeordnet sind. Das Sensorgehäuse kann hierbei in etwa mittig (betrachtet in einer Querschnittsebene senkrecht zu einer von proximal nach distal gerichteten Längserstreckungsrichtung des Sensorgehäuses) zwischen den Bügelelementen gehalten sein und über die Bügelelemente in einer implantierten Stellung derart an umliegenden Gefäßwandungen abgestützt sein, dass das Sensorgehäuse in etwa mittig in dem Gefäß gehalten wird.

Denkbar ist jedoch auch, dass die Bügelelemente unterschiedlich gestaltet sind und das Sensorgehäuse in implantierter Stellung nicht mittig in einem Gefäß abgestützt ist, sondern beispielsweise angenähert an oder gar in Kontakt mit einer Gefäßwandung.

Der der Erfindung zugrunde liegende Gedanke soll nachfolgend anhand der in den Figuren dargestellten Ausführungsbeispiele näher erläutert werden. Es zeigen:
- Fig. 1: eine Ansicht eines Ausführungsbeispiels einer Kathetereinrichtung mit einer mit der Kathetereinrichtung gekoppelten Sensoreinrichtung;
- Fig. 2: eine gesonderte Ansicht der Sensoreinrichtung;
- Fig. 3: eine Ansicht der Sensoreinrichtung an einer Kopplungseinrichtung der Kathetereinrichtung;
- Fig. 4: eine Ansicht der Sensoreinrichtung an der Kopplungseinrichtung, in einer gekoppelten Stellung;
- Fig. 5: eine Ansicht der Sensoreinrichtung an der Kopplungseinrichtung, nach Betätigen eines Verriegelungselements der Kopplungseinrichtung zum Freigeben der Sensoreinrichtung;
- Fig. 6: eine Seitenansicht der Kopplungseinrichtung;
- Fig. 7: eine Draufsicht auf die Kopplungseinrichtung;
- Fig. 8: eine Schnittansicht durch die Kopplungseinrichtung, in der gekoppelten Stellung;
- Fig. 9: eine Schnittansicht durch die Kopplungseinrichtung, in der Freigabestellung;
- Fig. 10A-10J: Ansichten der Kathetereinrichtung in Zusammenwirken mit einer Schleuse zum Platzieren der Sensoreinrichtung im Gefäß eines Patienten; und
- Fig. 11 und 12: eine weitere Ausführungsform der Sensoreinrichtung an der Kopplungseinrichtung in Anlehnung an die Fig. 4 und 5.

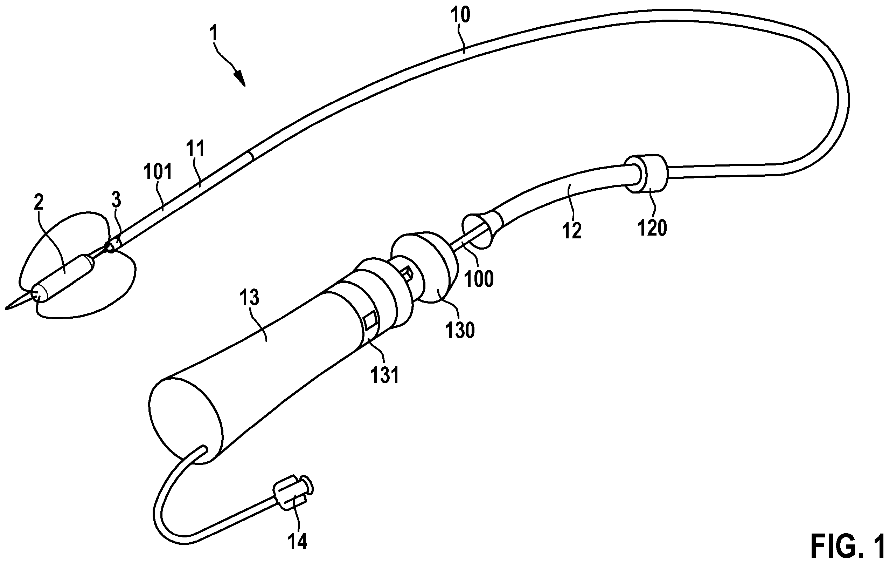

Eine in Fig. 1 dargestellte Kathetereinrichtung 1 weist einen Schaft 10 auf, der an einem proximalen Ende 100 mit einem Griff 13 verbunden ist und an einem distalen Ende 101 eine Kopplungseinrichtung 3 zur Kopplung mit einer Sensoreinrichtung 2 aufweist. Die Kathetereinrichtung 1 dient zum Implantieren der Sensoreinrichtung 2 in einem Gefäß eines Patienten, zum Beispiel im menschlichen Herzen oder in einem anderen Blutgefäß, um die beispielsweise als Drucksensor, Temperatursensor, Sauerstoffsensor oder Flusssensor ausgebildete Sensoreinrichtung 2 zu implantieren, beispielsweise zur Verwendung im Rahmen einer Heimüberwachung ("Home Monitoring") eines schwer kranken Patienten.

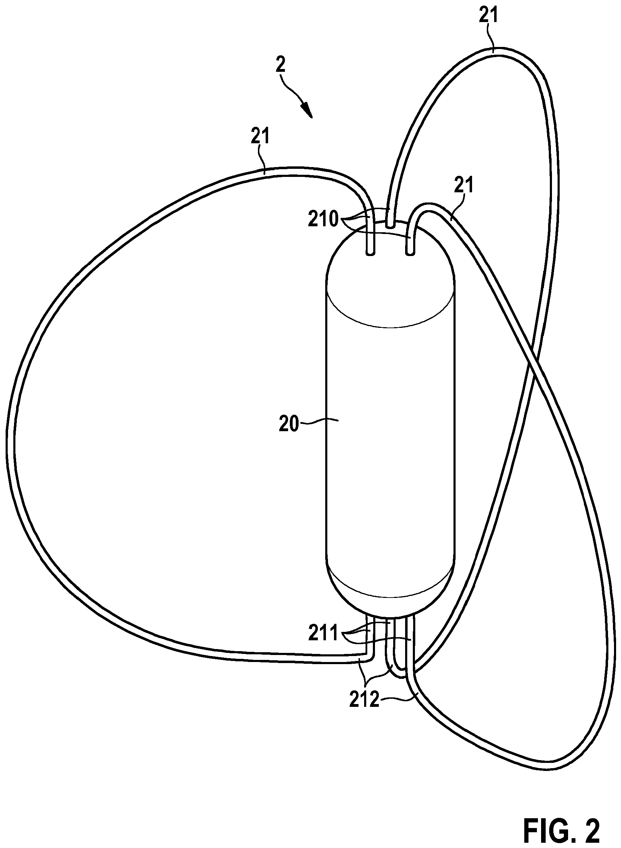

Die Sensoreinrichtung 2, dargestellt in einer gesonderten Ansicht in Fig. 2, weist ein Sensorgehäuse 20 auf, das beispielsweise eine Länge von ca. 15-20 mm und einen Durchmesser von ca. 3-5 mm aufweisen kann und elektrische und elektronische Komponenten der Sensoreinrichtung 2 einfasst. So kann die Sensoreinrichtung 2 beispielsweise eine eigene Energieversorgung, insbesondere eine Batterie, eine geeignete Sensorik, einen Prozessor sowie eine Kommunikationseinrichtung zur telemetrischen Datenübertragung aufweisen, um aufgenommene Sensorsignale vorzuverarbeiten und an externe Einrichtungen zu übertragen.

Die Sensoreinrichtung 2 ist, bei dem dargestellten Ausführungsbeispiel, als atraumatischer Sensor ausgebildet, der gefäßwandschonend in einem Patienten implantiert werden kann.

Die Sensoreinrichtung 2 weist Bügelelemente 21, jeweils gefertigt aus einem superelastischen Draht, auf, die gleichverteilt um das Sensorgehäuse 20 angeordnet sind und zur Abstützung des Sensorgehäuses 20 gegenüber umliegenden Gefäßwandungen dienen, wenn die Sensoreinrichtung 2 in einen Patienten implantiert ist. Die Bügelelemente 21 sind jeweils mit einem Ende 210 mit einem distalen Ende des Sensorgehäuses 20 und mit ihrem anderen Ende 211 mit einem proximalen Ende des Sensorgehäuses 20 verbunden, wie dies aus Fig. 2 ersichtlich ist.

Die Bügelelemente 21 können, bei dem dargestellten Ausführungsbeispiel, auch als Antennen zur Kommunikation mittels Telemetrie dienen.

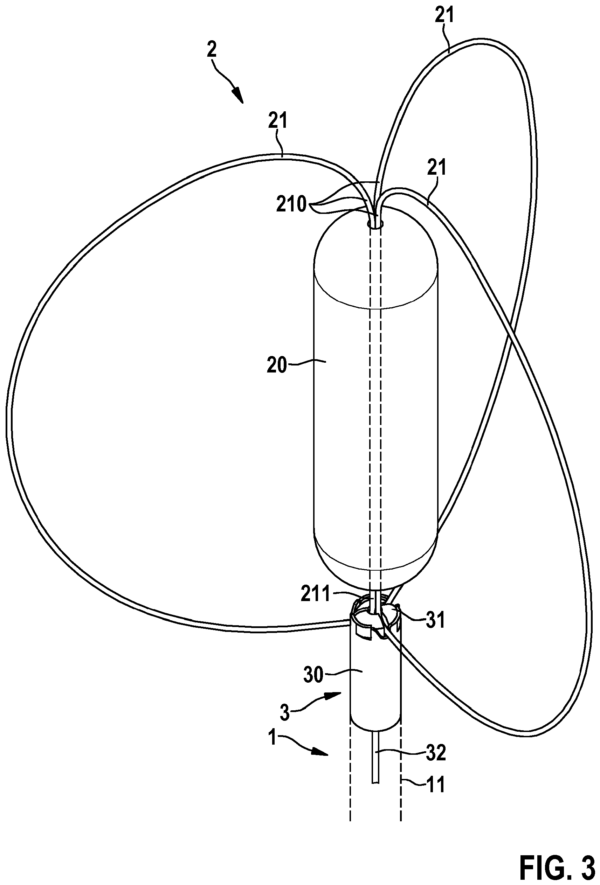

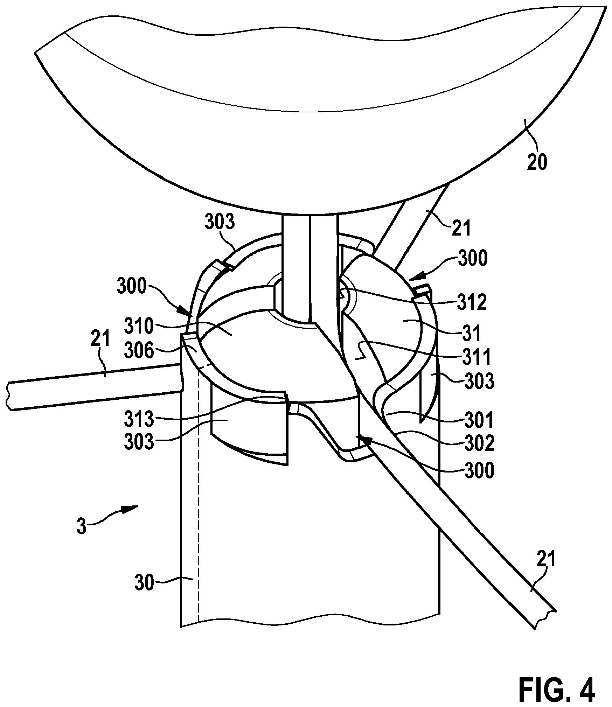

Die Kopplungseinrichtung 3 der Kathetereinrichtung 1 dient dazu, eine (lösbare) Verbindung zwischen der Kathetereinrichtung 1 und der Sensoreinrichtung 2 herzustellen, um die Sensoreinrichtung 2 unter Verwendung der Kathetereinrichtung 1 in einem Patienten zu implantieren. Wie aus Fig. 3 und 4 ersichtlich, stellt die Kopplungseinrichtung 3 in einer gekoppelten Stellung eine Verbindung zwischen der Kathetereinrichtung 1 und der Sensoreinrichtung 2 dadurch her, dass die Bügelelemente 21 der Sensoreinrichtung 2 über proximal geformte Ankerpunkte 212 an den Bügelelementen 21 (siehe Fig. 2) an der Kopplungseinrichtung 3 gehalten sind. Die Bügelelemente 21 können bei bestehender Kopplung entfaltet sein, also sich in einem Zustand befinden, in dem sie in implantiertem Zustand in einem menschlichen Gefäß einliegen.

Der Schaft 10 weist im Bereich seines distalen Endes 101 ein deflektierbares Schaftsegment 11 auf, das ausgelenkt werden kann, um auf diese Weise die Kathetereinrichtung 1 auch durch gewundene Gefäße hindurchführen zu können. Die Kopplungseinrichtung 3 ist an dem deflektierbaren Schaftsegment 11 angeordnet, sodass bei Auslenkung des Schaftsegments 11 eine mit der Kopplungseinrichtung 3 verbundene Sensoreinrichtung 2 gemeinsam mit dem Schaftsegment 11 ausgelenkt wird.

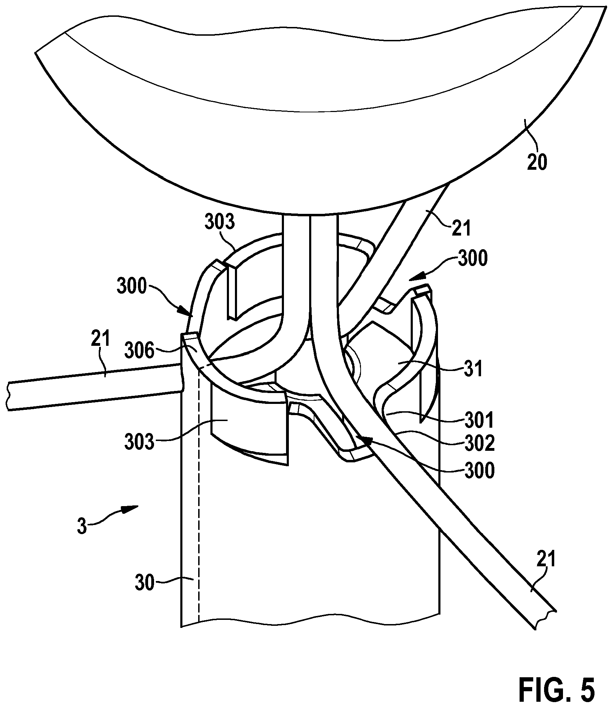

Die Kopplungseinrichtung 3 weist ein Kopplungselement 30 in Form einer Hülse auf, die fest mit dem Schaftsegment 11 und darüber mit dem Schaft 10 verbunden, zum Beispiel verklebt ist. Innerhalb des Kopplungselements 30 ist ein Verriegelungselement 31 aufgenommen, das zu dem Kopplungselement 30 verstellbar ist, um die Sensoreinrichtung 2 aus der gekoppelten Stellung gemäß Fig. 4 freizugeben und somit ein Lösen der Sensoreinrichtung 2 von der Kopplungseinrichtung 3 und somit von der Kathetereinrichtung 1 zu ermöglichen, wie dies in Fig. 5 dargestellt ist.

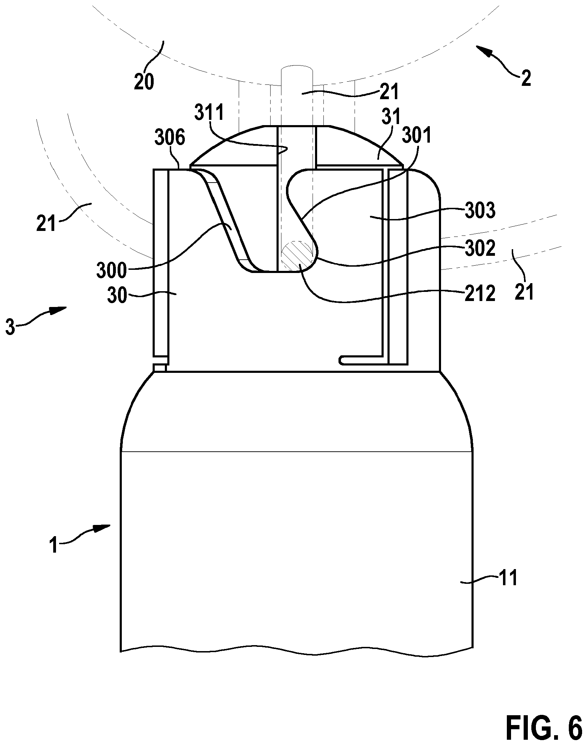

Die Kopplungseinrichtung 3 ist in unterschiedlichen Ansichten in Fig. 6 bis 9 dargestellt. An einem von dem Schaft 10 abliegenden, distalen Rand 306 sind Aussparungen 300 in das Kopplungselement 30 eingeformt, die jeweils einen Eingriffsabschnitt 302 nach Art eines nach außen hin durch einen Sperrabschnitt 301 begrenzten Hinterschnitts ausbilden. Ist die Sensoreinrichtung 2 mit der Kopplungseinrichtung 3 gekoppelt, so liegt ein jedes Bügelelement 21 mit einem Ankerpunkt 212 in einem der Eingriffsabschnitte 302 ein und ist darüber formschlüssig an dem Kopplungselement 30 gehalten.

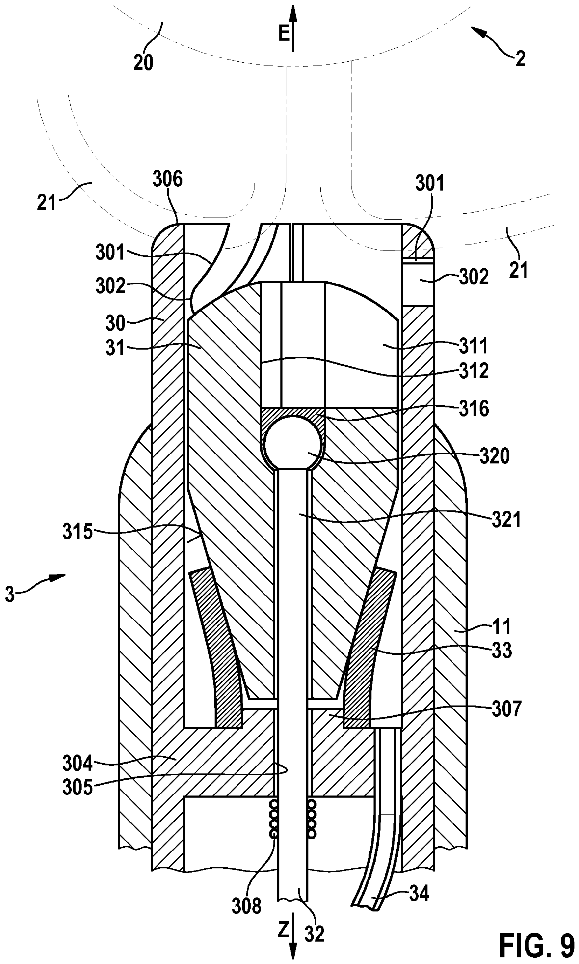

Der Sperrabschnitt 301 springt quer zu einer Trennrichtung E (siehe Fig. 9) in den Bereich der jeweils zugeordneten Aussparung 300 vor. Der Übergang zwischen dem Sperrabschnitt 301 und dem durch den Sperrabschnitt 301 begrenzten, zurückgesetzten Eingriffsabschnitt 302 ist hierbei zur Trennrichtung E schrägt erstreckt, wie dies insbesondere auch Fig. 6 ersichtlich ist.

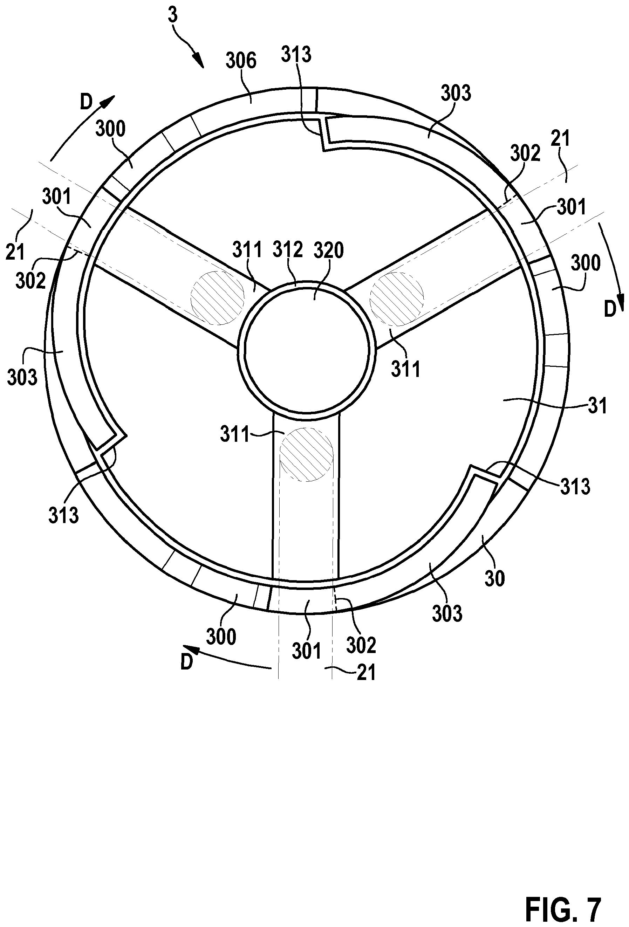

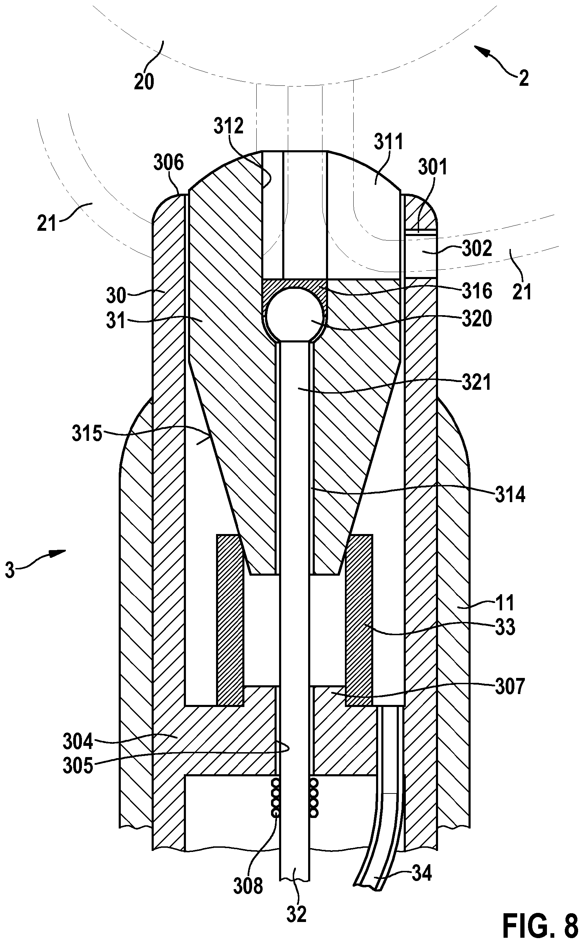

Das Verriegelungselement 31 ist entlang einer Betätigungsrichtung Z linear innerhalb des Kopplungselements 30 verstellbar, wie dies im Übergang von Fig. 4 hin zu Fig. 5 oder von Fig. 8 hin zu Fig. 9 ersichtlich ist. Das Verriegelungselement 31 ist in dem Kopplungselement 30 geführt und weist einen Körper 310 auf, an dem an einer distalen, nach außen weisenden Seite sternförmig zueinander angeordnete Eingriffsnuten 311 geformt sind, die zentral in eine Öffnung 312 münden, wie dies beispielsweise aus Fig. 4 und Fig. 7 ersichtlich ist. In der gekoppelten Stellung greift jedes Bügelelement 21 der Sensoreinrichtung 2 in eine der Eingriffsnuten 311 ein, wobei eine jede Eingriffsnut 311 mit einem zugeordneten Eingriffsabschnitt 302 des Kopplungselements 30 fluchtet und somit durch Eingriff des jeweiligen Bügelelements 21 in die Eingriffsnut 311 das Bügelelement 21 in dem Eingriffsabschnitt 302 gesperrt ist, wie dies zum Beispiel aus einer Zusammenschau von Fig. 4 und Fig. 6 ersichtlich ist.

Im deflektierten Zustand des Katheters könnte ein Zug am Zugelement 32 erst zu einer Verstärkung der Deflektion bzw. zu einem Positionierungsfehler führen, anstatt das Verriegelungselement 31 unmittelbar zurück zu ziehen und die Sensorreinrichtung 2 frei zu geben. Dies verhindert eine Kompressionsfeder 308, da sie bei Zug am Zugelement 32 als stabilisierendes Widerlager gegen den Bodenabschnitt 304 wirkt. Das Verriegelungselement 31 wird dadurch unmittelbar ohne Positionierungsfehler zurückgezogen.

Wird das Verriegelungselement 31 in die Betätigungsrichtung Z verstellt, so werden die Eingriffsnuten 311 aus dem Bereich der Eingriffsabschnitte 302 entfernt. Die Bügelelemente 21 gelangen dadurch, wie dies aus Fig. 5 ersichtlich ist, außer Eingriff von den Eingriffsnuten 311, sodass die Bügelelemente 21 nicht mehr zu den Eingriffsabschnitten 302 gesperrt sind und in eine Löserichtung D (siehe Fig. 7) durch Auflaufen auf den schrägen Übergang zwischen den Eingriffsabschnitten 302 und den Sperrabschnitten 301 außer Eingriff von den Eingriffsabschnitten 302 gebracht werden können. Die Sensoreinrichtung 2 kann somit entlang der Trennrichtung E (siehe Fig. 9) von der Kopplungseinrichtung 3 entfernt werden, ohne dass die Bügelelemente 21 in den Aussparungen 300 verhaken können.

Das Verriegelungselement 31 ist an dem Kopplungselement 30 entlang der Betätigungsrichtung Z geführt und insbesondere in der gekoppelten Stellung (Fig. 4) verdrehsicher zu dem Kopplungselement 30 abgestützt. Dazu weist das Kopplungselement 30 radial nach innen vorspringende Anschlagelemente 303 auf, die jeweils mit einem zugeordneten Anschlag 313 an der äußeren Umfangsfläche des Körpers 310 des Verriegelungselements 31 zusammenwirken derart, dass ein Verdrehen des Verriegelungselements 31 insbesondere in die Löserichtung D zu dem Kopplungselement 30 verhindert ist.

Das Verriegelungselement 31 ist mit einem Zugelement 32 verbunden, das mit einem kugelförmigen Verbindungselement 320 in der zentralen Öffnung 312 des Verriegelungselements 31 einliegt und sich mit einen Abschnitt 321 durch einen an die Öffnung 312 anschließenden, längserstreckten, verjüngten Kanal 314 des Verriegelungselements 31 hindurch erstreckt, wie dies aus Fig. 8 und 9 ersichtlich ist. Das Zugelement 32 verläuft durch eine Öffnung 305 in einem Bodenabschnitt 304 des Kopplungselements 30 hindurch und erstreckt sich entlang des Schafts 10 bis hin zu dem Griff 13 und ist aufseiten des Griffs 13 mit einem Betätigungselement 130 gekoppelt.

Das Zugelement 32 ist über das Verbindungselement 320 fest mit dem Verriegelungselement 31 verbunden. Das kugelförmige Verbindungselement 320 ist hierbei über eine Klebemasse 316 mit dem Verriegelungselement 31 verklebt und dadurch fest verbunden, wobei die Klebemasse 316 zudem eine Abdichtung des Kanals 314 distal nach außen hin bereitstellt.

Zwischen dem Verriegelungselement 31 und dem Bodenabschnitt 304 des Kopplungselements 30 ist ein hülsenförmiges (in einem entspannten Zustand zylindrisches) elastisches Element 33 angeordnet, in das ein an dem Körper 310 des Verriegelungselements 31 geformter Konusabschnitt 315 eingreift und das elastisch vorspannend sowie dichtend zwischen dem Verriegelungselement 31 und dem Bodenabschnitt 304 des Kopplungselements 30 wirkt. Das elastische Element 33, gefertigt aus einem elastischen, zum Beispiel einem elastomeren Material, ist (vorteilhafterweise mit umfänglicher Vorspannung) an einen erhabenen Abschnitt 307 des Bodenabschnitts 304 angesetzt und umgibt die in dem Bodenabschnitt 304 geformte, das Zugelement 32 führende Öffnung 305 derart, dass die Öffnung 305 nach außen hin feuchtigkeitsdicht abgedichtet ist. Insbesondere wirkt das elastische Element 33 derart zwischen dem Verriegelungselement 31 und dem Bodenabschnitt 304, dass Feuchtigkeit nicht von außen in den Bereich des Zugelements 32 und darüber über die Öffnung 305 in das Innere des Schafts 10 gelangen kann.

Das elastische Element 33 dient zudem zur elastischen Rückstellung des Verriegelungselements 31 nach erfolgter Betätigung. So weitet der Konusabschnitt 315 bei einem Verstellen des Verriegelungselements 31 in die Betätigungsrichtung Z das elastische Element 33 (siehe Fig. 8 im Übergang hin zu Fig. 9). Lassen an dem Zugelement 32 bewirkte Zugkräfte nach, bewirkt diese elastische Verformung des elastischen Elements 33 eine Rückstellkraft auf das Verriegelungselement 31 entgegen der Betätigungsrichtung Z in Richtung der in Fig. 8 dargestellten Ausgangsstellung, sodass das Verriegelungselement 31 selbsttätig in die der gekoppelten Stellung zugeordnete Ausgangsstellung zurückgestellt wird.

Diese Rückstellung kann hierbei auch das Trennen der Sensoreinrichtung 2 von der Kopplungseinrichtung 3 unterstützen, indem das Verriegelungselement 31 mit seiner distalen Stirnseite auf die Bügelelemente 21 einwirkt und dadurch die Sensoreinrichtung 2 in die Trennrichtung E von der Kopplungseinrichtung 3 weg drückt.

Am Bodenabschnitt 304 des Kopplungselements 30 mündet eine Spülleitung 34, die dazu dient, einen Innenraum des Kopplungselements 30 zu spülen. Über die Spülleitung 34 können - insbesondere in der gekoppelten Stellung der Sensoreinrichtung 2 - Zwischenräume zwischen dem Verriegelungselement 31, dem Kopplungselement 30 und den Bügelelementen 21 der Sensoreinrichtung 2 zum Beispiel mit einer Kochsalzlösung gespült und zudem auch entlüftet werden.

Die Spülleitung 34 ist mit einem Spülanschluss 14 am Griff 13 (siehe Fig. 1) verbunden, sodass eine Spülflüssigkeit über den Spülanschluss 14 hin zu der Spülleitung 34 eingeleitet werden kann.

Über das Betätigungselement 130 des Griffs 13 können, bei dem dargestellten Ausführungsbeispiel, das Verriegelungselement 31 der Kopplungseinrichtung 3 sowie das deflektierbare Schaftsegment 11 des Schafts 10 betätigt werden. Das Betätigungselement 130 kann am Griff 13 verdreht werden, um auf diese Weise eine Zugkraft auf das Zugelement 32 zu bewirken und das Verriegelungselement 31 in die Betätigungsrichtung Z zu dem Kopplungselement 30 zu verstellen. Zudem kann das Betätigungselement 130 linear an dem Griff 13 verschoben werden, um auf diese Weise, zum Beispiel über ein zugeordnetes, in dem Schaft 10 geführtes Zugseil, das Schaftsegment 11 auszulenken und somit die Kathetereinrichtung 1 durch gewundene Gefäße zu führen.

Am Griff 13 ist, bei dem dargestellten Ausführungsbeispiel, ein Sicherungselement 131 angeordnet, das in einer Sicherungsstellung ein Verdrehen des Betätigungselements 130 und somit ein (unbeabsichtigtes) Lösen der Sensoreinrichtung 2 von der Kopplungseinrichtung 3 verhindert (ein lineares Verschieben des Betätigungselements 130 zum Betätigen des deflektierbaren Schaftsegments 11 aber zulässt). Das Sicherungselement 131 kann entfernt werden, um das Betätigungselement 130 verdrehen zu können.

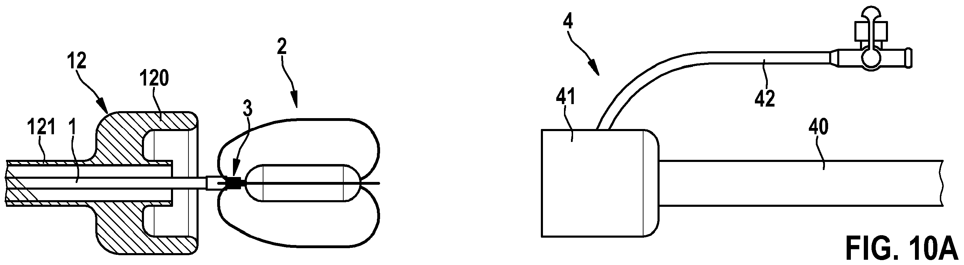

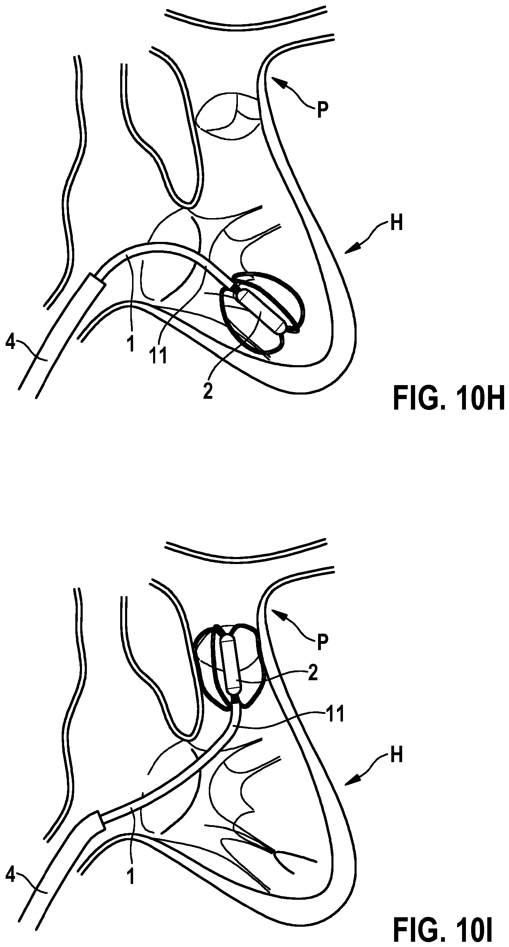

Fig. 10A bis 10J zeigen ein mögliches Vorgehen zum Implantieren einer Sensoreinrichtung 2 unter Verwendung der Kathetereinrichtung 1 in einem Patienten.

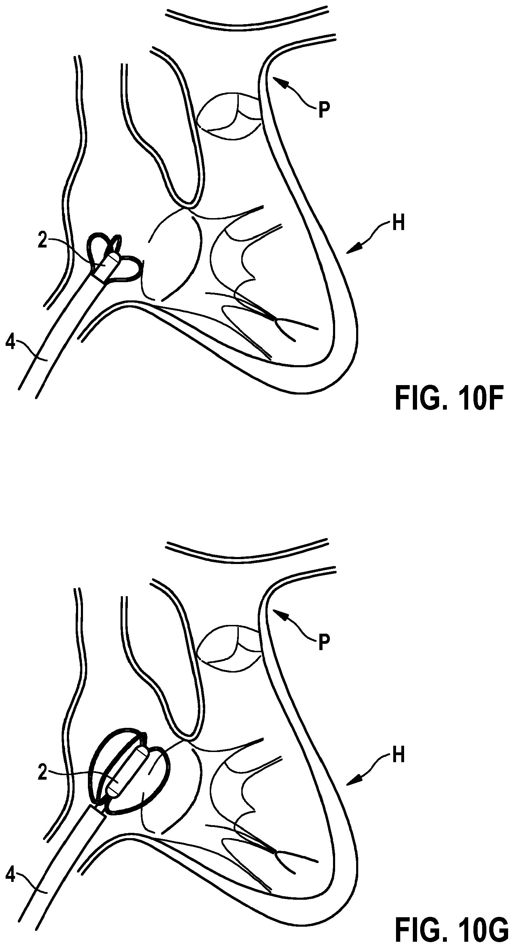

Wie aus Fig. 1 ersichtlich, ist am Schaft 10 der Kathetereinrichtung 1 eine Schleuseneinführhilfe 12 angeordnet, die dazu dient, die Kathetereinrichtung 1 mit der gekoppelten Sensoreinrichtung 2 in eine Schleuse 4 einzuführen, wie dies in Fig. 10A bis 10E dargestellt ist. Die Schleuse 4 dient als Zugang zum Patienten und ist so gelegt, dass mittels der Schleuse 4 die Kathetereinrichtung 1 mit der gekoppelten Sensoreinrichtung 2 beispielsweise bis hin zum menschlichen Herzen H geführt werden kann, wie dies aus Fig. 10F bis 10J ersichtlich ist.

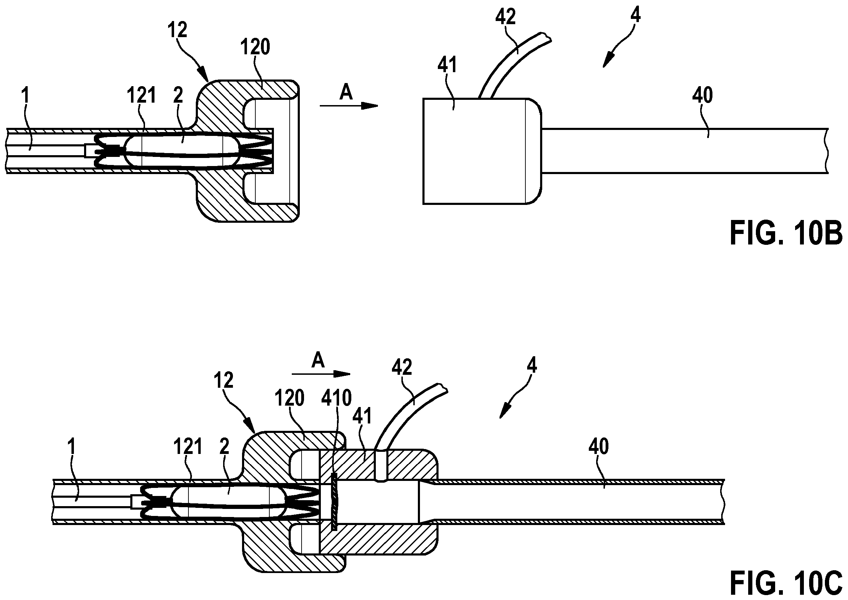

Die Schleuse 4 ist von außerhalb des Patienten zugänglich. Zum Einführen der Kathetereinrichtung 1 mit der gekoppelten Sensoreinrichtung 2 wird die Sensoreinrichtung 2 zunächst, wie dies aus Fig. 10A im Übergang hin zu Fig. 10B ersichtlich ist, auf Seiten eines Ansetzstücks 120 in die Schleuseneinführhilfe 12 eingezogen, sodass die Bügelelemente 21 der Sensoreinrichtung 2 eingefaltet werden und die Sensoreinrichtung 2 innerhalb eines Aufnahmeschafts 121 der Schleuseneinführhilfe 12 zu liegen kommt (Fig. 10B).

Am Eingang des Einführstutzens 41 weist die Schleuse 4 ein Dichtelement 410 (Dichtlippen) auf, durch das hindurch die Kathetereinrichtung 1 bei Einführen in die Schleuse 4 tritt und das einen Übergang nach außen hin abdichtet.

Nunmehr wird die Schleuseneinführhilfe 12 mit der darin aufgenommenen Sensoreinrichtung 2 mit dem Ansetzstück 120 an einen Einführstutzen 41 der Schleuse 4 angesetzt, sodass ein innerer Kanal des Aufnahmeschafts 121 der Schleuseneinführhilfe 12 mit einem Schleusenschaft 40 der Schleuse 4 fluchtet (Fig. 10C). Die nach distal in runden Schlaufen gebogenen Bügelelemente 21 schieben das Dichtelement 410 des Schleusenventils schonend beiseite. Nach Durchgang der Sensoreinrichtung 2 umschließt das Dichtelement 410 die vorwärtsgeschobenen Schaftsegmente dicht.

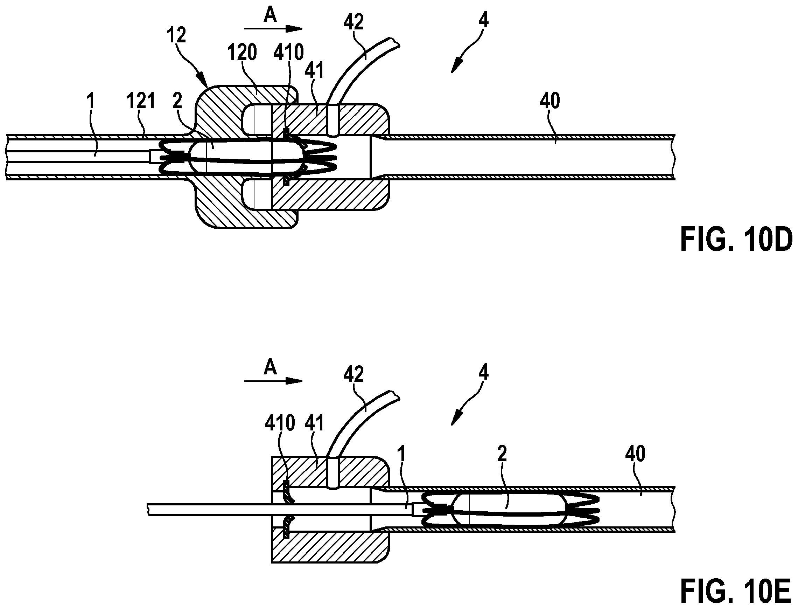

Durch Einschieben der Kathetereinrichtung 1 mit der gekoppelten Sensoreinrichtung 2 in eine Einführrichtung A in den Schleusenschaft 40 wird nunmehr die Sensoreinrichtung 2 in den Schleusenschaft 40 verbracht (Fig. 10D und 10E) und kann somit durch die Schleuse 4 hindurch bis in das Gefäß, in dem die Sensoreinrichtung 2 zu platzieren ist (im beispielhaft dargestellten Fall dem menschlichen Herzen H), geführt werden, wie dies aus Fig. 10F ersichtlich ist.

Im beispielhaft dargestellten Fall soll die Sensoreinrichtung 2 im Bereich der Pulmonalarterie P platziert werden. Die Kathetereinrichtung 1 mit der distal gekoppelten Sensoreinrichtung 2 wird dazu ausgangsseitig der Schleuse 4 durch das menschliche Herz H hindurchgeführt und - unter Auslenkung des deflektierbaren Schaftsegments 11 - bis in den Bereich der Pulmonalarterie P geführt, bis ein bestimmungsgemäßer Implantationsort der Sensoreinrichtung 2 erreicht ist (siehe Fig. 10G bis Fig. 10J).

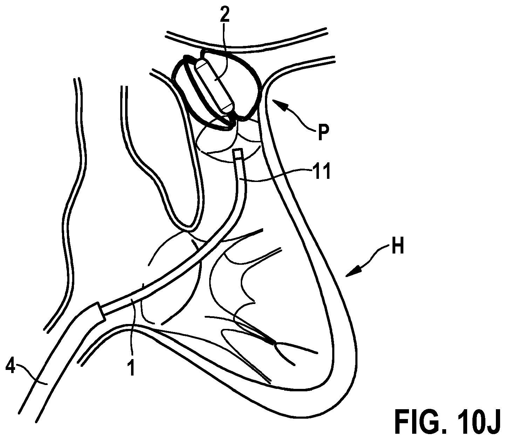

Insbesondere kann die Sensoreinrichtung 2 im rechten Vorhof aus der Schleuse 4 austreten (Fig. 10F). Beim Austreten aus der Schleuse 4 entfalten sich die Bügelelemente 21, wie dies im Übergang von Fig. 10F hin zu Fig. 10G ersichtlich ist. In diesem Zustand kann die Sensoreinrichtung 2 überprüft werden (beispielsweise mittels geeigneter Bildgebungsverfahren), wobei die Sensoreinrichtung 2 zurückgezogen werden kann, wenn ein Fehler oder eine Beschädigung an der Sensoreinrichtung 2 festgestellt wird. Mittels der Kathetereinrichtung 1 wird die Sensoreinrichtung 2 nunmehr beispielsweise durch die Trikuspidalklappe in den rechten Ventrikel manövriert (Fig. 10H), und die Sensoreinrichtung 2 wird unter Auslenkung des deflektierbaren Schaftsegments 11 an der anterioren Ventrikelinnenwand entlang in die Pulmonalklappe eingeführt (Fig. 101). Die Bügelelemente 21an der Sensoreinrichtung 2 verhindern, dass sich die Kathetereinrichtung hinter die Sehnenfäden (Chordae tendineae) der Trikuspidalklappe schieben und diese ggf. beschädigen. Nach Überwindung der Pulmonalklappe wird die Sensoreinrichtung 2 an die gewünschte Position in der Pulmonalarterie P vorgebracht (Fig. 10J). Ist ein bestimmungsgemäße Ort erreicht, kann die Kopplungseinrichtung 3 entriegelt werden, um die Sensoreinrichtung 2 abzukoppeln. Die Sensoreinrichtung 2 verbleibt somit an ihrem Bestimmungsort. Die Kathetereinrichtung 1 kann zurückgezogen werden.

Wird, beispielsweise mittels Bildgebung, festgestellt, dass die Sensoreinrichtung 2 nicht korrekt platziert worden ist, so kann mittels der Kathetereinrichtung 1 und der daran angeordneten Kopplungseinrichtung 3 die Sensoreinrichtung 2 erneut gegriffen werden, um die Lage der Sensoreinrichtung 2 zu korrigieren.

Mittels der Kathetereinrichtung 1 kann eine implantierte Sensoreinrichtung 2 ggf. wieder aus dem menschlichen Körper entfernt werden.

Am Einführstutzen 41 ist zudem eine Spülleitung 42 angeordnet, die zum Spülen des Schleusenschafts 40 bei Einführen der Kathetereinrichtung 1 mit der gekoppelten Sensoreinrichtung 2 durch die Schleuse 4 hindurch dient.

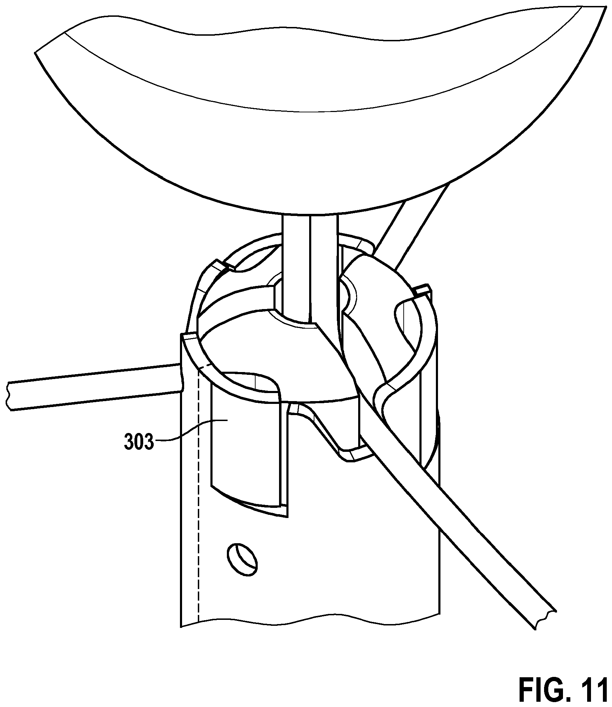

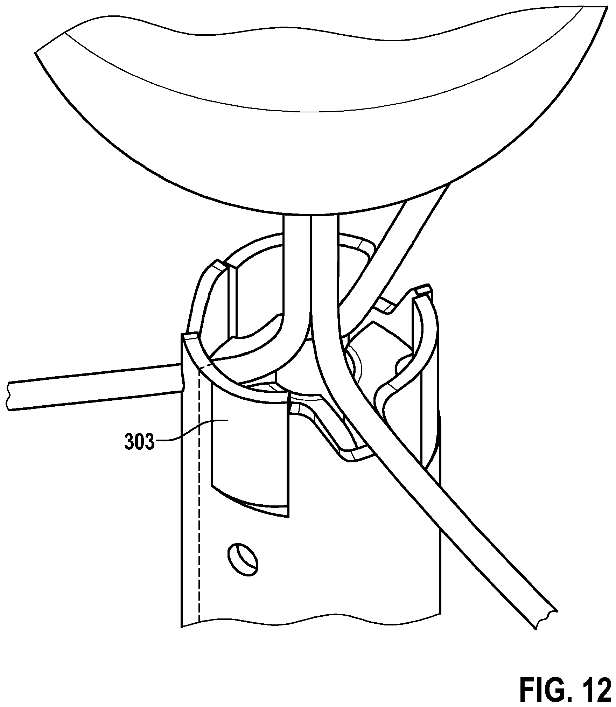

Fig. 11 und 12 zeigen eine weitere Ausführungsform der Sensoreinrichtung an der Kopplungseinrichtung in Anlehnung an die Fig. 4 und 5. Eine Nut zwischen dem Anschlagelement 303 und dem gegenüberliegenden Abschnitt des Gehäuses ist breiter und länger als bei der in Fig. 4 und 5 gezeigten Ausführungsform. Ansonsten entsprechen alle Komponenten den bereits in Fig. 4 und 5 dargestellten und oben beschriebenen Komponenten.

Der der Erfindung zugrunde liegende Gedanke ist nicht auf die vorangehend geschilderten Ausführungsbeispiele beschränkt, sondern lässt sich auch in anderer Weise verwirklichen.

Eine Sensoreinrichtung der beschriebenen Art kann mehr oder weniger als drei Bügelelemente aufweisen. So ist beispielsweise denkbar, dass die Sensoreinrichtung lediglich zwei Bügelelemente aufweist. Auch ist denkbar, dass die Sensoreinrichtung mehr als drei Bügelelemente, beispielsweise vier oder fünf Bügelelemente, aufweist.

Mittels der Bügelelemente kann das Sensorgehäuse zentral zwischen den Bügelelementen und somit zentral in einem Gefäß gehalten sein. Denkbar ist aber auch, dass die Bügelelemente das Sensorgehäuse dezentral, also außermittig, beispielsweise einer Gefäßwand angenähert, halten.

Eine solche Sensoreinrichtung kann als Drucksensor, Temperatursensor, Sauerstoffsensor oder Flusssensor oder auch als anderer Sensor ausgebildet sein und weist eine entsprechende Sensorik auf. Die Sensoreinrichtung kann hierbei über einen längeren Zeitraum im implantierten Zustand in autarker Weise funktionieren, um erfasste Sensordaten, beispielsweise im Rahmen einer Heimüberwachung, zum Beispiel mittels Telemetrie an eine externe Einrichtung zu übertragen.

### Liste der Bezugszeichen

- 1: Kathetereinrichtung
- 10: Schaft
- 100: Proximales Ende
- 101: Distales Ende
- 11: Abschnitt (deflektierbares Schaftsegment)
- 12: Schleuseneinführhilfe
- 120: Ansetzstück
- 121: Aufnahmeschaft
- 13: Griff
- 130: Betätigungselement
- 131: Sicherungselement
- 14: Spülanschluss
- 2: Sensoreinrichtung
- 20: Sensorgehäuse
- 21: Bügelelement (Drahtbügel)
- 210,211: Ende
- 212: Ankerpunkt
- 3: Kopplungseinrichtung
- 30: Kopplungselement (Kopplungshülse)
- 300: Aussparung
- 301: Sperrabschnitt
- 302: Eingriffsabschnitt
- 303: Anschlagelement
- 304: Bodenabschnitt
- 305: Öffnung
- 306: Rand
- 307: Erhabener Abschnitt
- 308: Kompressionswendel
- 31: Verriegelungselement
- 310: Körper
- 311: Eingriffsnut
- 312: Öffnung
- 313: Anschlag
- 314: Kanal
- 315: Konusabschnitt
- 316: Klebemasse
- 32: Zugelement
- 320: Verbindungselement
- 321: Abschnitt
- 33: Elastisches Element
- 34: Spülleitung
- 4: Schleuse
- 40: Schleusenschaft
- 41: Einführstutzen
- 410: Dichtelement
- 42: Spülleitung
- A: Einführrichtung
- D: Löserichtung
- E: Trennrichtung
- Z: Betätigungsrichtung

## Patentansprüche

1. Kathetereinrichtung (1) zum Platzieren einer Sensoreinrichtung (2), mit einem Schaft (10) und einer an dem Schaft (10) angeordneten Kopplungseinrichtung (3) zum Koppeln der Kathetereinrichtung (1) mit der zumindest ein Bügelelement (21) aufweisenden Sensoreinrichtung (2), wobei die Kopplungseinrichtung (3) ein Kopplungselement (30) und ein zu dem Kopplungselement (30) verstellbares Verriegelungselement (31) aufweist, wobei das Kopplungselement (30) zumindest einen Eingriffsabschnitt (302) aufweist, mit dem das zumindest eine Bügelelement (21) der Sensoreinrichtung (2) in Eingriff bringbar ist, wobei das Verriegelungselement (31) ausgebildet ist, in einer gekoppelten Stellung das zumindest eine Bügelelement (21) an dem zumindest einen Eingriffsabschnitt (302) zu sperren, und zu dem Kopplungselement (30) verstellbar ist, um das zumindest eine Bügelelement (21) zum Lösen von dem zumindest einen Eingriffsabschnitt (302) freizugeben,
**gekennzeichnet dadurch, dass**
die Kopplungseinrichtung (3) ein an dem Kopplungselement (30) angeordnetes, zwischen dem Kopplungselement (30) und dem Verriegelungselement (31) wirkendes, elastisches Element (33) aufweist.

2. Kathetereinrichtung (1) nach Anspruch 1, wobei das Kopplungselement (30) fest mit einem Abschnitt (11) des Schafts (10) verbunden ist.

3. Kathetereinrichtung (1) nach Anspruch 1 oder 2, wobei das Verriegelungselement (31) entlang einer Betätigungsrichtung (Z) linear zu dem Kopplungselement (30) verstellbar ist.

4. Kathetereinrichtung (1) nach Anspruch 3, wobei das Kopplungselement (30) als Hülse ausgebildet ist, in der das Verriegelungselement (31) entlang der Betätigungsrichtung (Z) geführt ist.

5. Kathetereinrichtung (1) nach einem der vorangehenden Ansprüche, wobei das Kopplungselement (30) an einem von dem Schaft (10) abliegenden Rand (306) zumindest eine Aussparung (300) aufweist, an der der zumindest eine Eingriffsabschnitt (302) geformt ist.

6. Kathetereinrichtung (1) nach einem der vorangehenden Ansprüche, wobei das Verriegelungselement (31) zumindest eine Eingriffsnut (302) zum Aufnehmen des zumindest einen Bügelelements (21) in der gekoppelten Stellung aufweist.

7. Kathetereinrichtung (1) nach einem der vorangehenden Ansprüche, wobei die Kopplungseinrichtung (3) ein mit dem Verriegelungselement (31) verbundenes, entlang des Schafts (10) erstrecktes Zugelement (32) zum Verstellen des Verriegelungselements (31) aufweist.

8. Kathetereinrichtung (1) nach Anspruch 7, ferner aufweisend einen Griff (13), der an einem der Kopplungseinrichtung (3) abgewandten Ende des Schafts (10) angeordnet ist, wobei der Griff (13) ein Betätigungselement (130) zum Betätigen des Zugelements (32) aufweist.

9. Kathetereinrichtung (1) nach Anspruch 1, wobei das Verriegelungselement (31) einen Körper (310) und einen an dem Körper (310) geformten Konusabschnitt (315) zum Wechselwirken mit dem elastischen Element (33) aufweist.

10. Kathetereinrichtung (1) nach Anspruch 1 oder 9, wobei das Kopplungselement (30) einen Bodenabschnitt (304) aufweist, an dem das elastische Element (33) angeordnet ist und dem das Verriegelungselement (31) bei einem Verstellen zum Lösen des zumindest einen Bügelelements (21) von dem zumindest einen Eingriffsabschnitt (302) angenähert wird.

11. Kathetereinrichtung (1) nach einem der vorangehenden Ansprüche, wobei der Schaft (10) ein deflektierbares Schaftsegment (11) aufweist, an dem die Kopplungseinrichtung (3) angeordnet ist.

12. System mit einer Kathetereinrichtung (1) nach einem der vorangehenden Ansprüche und einer mit der Kopplungseinrichtung (3) der Kathetereinrichtung (1) zu koppelnden Sensoreinrichtung (2).

13. System nach Anspruch 12, wobei die Sensoreinrichtung (2) ein Sensorgehäuse (20) und zumindest ein an dem Sensorgehäuse (20) angeordnetes Bügelelement (21) aufweist, das in der gekoppelten Stellung mit dem zumindest einen Eingriffsabschnitt (302) des Kopplungselements (30) in Eingriff steht.

14. System nach Anspruch 13, wobei das zumindest eine Bügelelement (21) mit einem ersten Ende (210) an einer distalen Seite des Sensorgehäuses (20) und mit einem zweiten Ende (211) mit einem der distalen Seite abgewandten, proximalen Seite des Sensorgehäuses (20) verbunden ist.

## Claims

1. A catheter device (1) for placing a sensor device (2), comprising a shaft (10) and a coupling device (3), arranged at the shaft (10), for coupling the catheter device (1) to the sensor device (2) comprising at least one bracket element (21), wherein the coupling device (3) comprises a coupling element (30) and a locking element (31) adjustable with respect to the coupling element (30), the coupling element (30) comprising at least one engagement section (302) with which the at least one bracket element (21) of the sensor device (2) can be brought in engagement, the locking element (31) being designed, in a coupled position, to block the at least one bracket element (21) at the at least one engagement section (302) and being adjustable with respect to the coupling element (30) so as to release the at least one bracket element (21) for detachment from the at least one engagement section (302),
**characterized in that**
the coupling device (3) has an elastic element (33) arranged on the coupling element (30) and acting between the coupling element (30) and the locking element (31).

2. The catheter device (1) according to claim 1, wherein the coupling element (30) is fixedly connected to a section (11) of the shaft (10).

3. The catheter device (1) according to claim 1 or 2, wherein the locking element (31) is linearly adjustable along an actuating direction (Z) with respect to the coupling element (30).

4. The catheter device (1) according to claim 3, wherein the coupling element (30) is designed as a sleeve, in which the locking element (31) is guided along the actuating direction (Z).

5. A catheter device (1) according to any one of the preceding claims, wherein the coupling element (30), at a rim (306) located away from the shaft (10), comprises at least one recess (300) at which the at least one engagement section (302) is formed.

6. A catheter device (1) according to any one of the preceding claims, wherein the locking element (31) comprises at least one engagement groove (302) for receiving the at least one bracket element (21) in the coupled position.

7. A catheter device (1) according to any one of the preceding claims, wherein the coupling device (3) comprises a pulling element (32), which is connected to the locking element (31) and extends along the shaft (10), for adjusting the locking element (31).

8. The catheter device (1) according to claim 7, further comprising a handle (13), which is arranged at an end of the shaft (10) which faces away from the coupling device (3), the handle (13) comprising an actuating element (130) for actuating the pulling element (32).

9. The catheter device (1) according to claim 1, wherein the locking element (31) comprises a body (310) and a conical section (315), formed at the body (310), for interacting with the elastic element (33).

10. The catheter device (1) according to claim 1 or 9, wherein the coupling element (30) comprises a bottom section (304), at which the elastic element (33) is arranged and to which the locking element (31) is moved closer during an adjustment for detachment of the at least one bracket element (21) from the at least one engagement section (302).

11. A catheter device (1) according to any one of the preceding claims, wherein the shaft (10) comprises a deflectable shaft segment (11), at which the coupling device (3) is arranged.

12. A system comprising a catheter device (1) according to any one of the preceding claims, and comprising a sensor device (2) to be coupled to the coupling device (3) of the catheter device (1).

13. The system according to claim 12, wherein the sensor device (2) comprises a sensor housing (20) and at least one bracket element (21), which is arranged at the sensor housing (20) and, in the coupled position, engages with the at least one engagement section (302) of the coupling element (30).

14. The system according to claim 13, wherein the at least one bracket element (21) is attached with a first end (210) to a distal side of the sensor housing (20), and with a second end (211) to a proximal side of the sensor housing (20) which faces away from the distal side.

## Revendications

1. Dispositif de cathéter (1) pour la mise en place d'un dispositif de capteur (2), avec une tige (10) et un dispositif de couplage (3) disposé sur la tige (10) pour le couplage du dispositif de cathéter (1) avec le dispositif de capteur (2) présentant au moins un élément d'étrier (21), le dispositif de couplage (3) présentant un élément de couplage (30) et un élément de verrouillage (31) déplaçable par rapport à l'élément de couplage (30), l'élément de couplage (30) présentant au moins une section d'engagement (302), avec laquelle l'au moins un élément en forme d'étrier (21) du dispositif de détection (2) peut être amené en prise, l'élément de verrouillage (31) étant conçu pour bloquer, dans une position couplée, l'au moins un élément en forme d'étrier (21) sur l'au moins une section de prise (302), et pouvant être déplacé par rapport à l'élément de couplage (30) afin de libérer l'au moins un élément en forme d'étrier (21) pour le libérer de l'au moins une section de prise (302),
**caractérisé en ce que**
le dispositif d'accouplement (3) comprend un élément élastique (33) disposé sur l'élément d'accouplement (30) et agissant entre l'élément d'accouplement (30) et l'élément de verrouillage (31).

2. Dispositif de cathéter (1) selon la revendication 1, dans lequel l'élément de couplage (30) est relié de manière fixe à une section (11) de la tige (10).

3. Dispositif de cathéter (1) selon la revendication 1 ou 2, dans lequel l'élément de verrouillage (31) est réglable linéairement par rapport à l'élément de couplage (30) le long d'une direction d'actionnement (Z).

4. Dispositif de cathéter (1) selon la revendication 3, dans lequel l'élément de couplage (30) est conçu comme un manchon dans lequel l'élément de verrouillage (31) est guidé le long de la direction d'actionnement (Z).

5. Dispositif de cathéter (1) selon l'une des revendications précédentes, dans lequel l'élément de couplage (30) présente, sur un bord (306) éloigné de la tige (10), au moins un évidement (300) sur lequel est formée la au moins une section d'engagement (302).

6. Dispositif de cathéter (1) selon l'une des revendications précédentes, dans lequel l'élément de verrouillage (31) comprend au moins une rainure d'engagement (302) pour recevoir ledit au moins un élément d'étrier (21) dans la position couplée.

7. Dispositif de cathéter (1) selon l'une des revendications précédentes, dans lequel le dispositif de couplage (3) présente un élément de traction (32) relié à l'élément de verrouillage (31) et s'étendant le long de la tige (10) pour déplacer l'élément de verrouillage (31).

8. Dispositif de cathéter (1) selon la revendication 7, présentant en outre une poignée (13) qui est disposée à une extrémité de la tige (10) opposée au dispositif de couplage (3), la poignée (13) présentant un élément d'actionnement (130) pour actionner l'élément de traction (32).

9. Dispositif de cathéter (1) selon la revendication 1, dans lequel l'élément de verrouillage (31) comprend un corps (310) et une partie conique (315) formée sur le corps (310) pour interagir avec l'élément élastique (33).

10. Dispositif de cathéter (1) selon la revendication 1 ou 9, dans lequel l'élément de couplage (30) comprend une partie inférieure (304) sur laquelle l'élément élastique (33) est disposé et dont l'élément de verrouillage (31) est approché lors d'un déplacement pour libérer l'au moins un élément d'étrier (21) de l'au moins une partie d'engagement (302).

11. Dispositif de cathéter (1) selon l'une des revendications précédentes, dans lequel la tige (10) comprend un segment de tige (11) pouvant être défléchi, sur lequel le dispositif de couplage (3) est disposé.

12. Système comprenant un dispositif cathéter (1) selon l'une des revendications précédentes et un dispositif capteur (2) à coupler au dispositif de couplage (3) du dispositif cathéter (1).

13. Système selon la revendication 12, dans lequel le dispositif de détection (2) comprend un boîtier de détection (20) et au moins un élément d'étrier (21) disposé sur le boîtier de détection (20) et qui, dans la position couplée, est en prise avec la au moins une partie d'engagement (302) de l'élément de couplage (30).

14. Système selon la revendication 13, dans lequel le au moins un élément d'étrier (21) est relié par une première extrémité (210) à un côté distal du boîtier de capteur (20) et par une deuxième extrémité (211) à un côté proximal du boîtier de capteur (20) opposé au côté distal.
